# EUROPEAN PATENT APPLICATION

(11) **EP 2 656 851 A1**
(43) Date of publication of application: **30.10.2013**
(21) Application number: 12305479.3
(22) Date of filing: 27.04.2012
(51) Int. Cl.: A61K 31/727, A61K 31/00, A61P 35/00, A61P 7/02

(54) **Semuloparin for improving the survival of patients with cancer**

(71) Applicant: Aventis Pharma S.A., 92160 Antony (FR)
(72) Inventor: The designation of the inventor has not yet been filed
(74) Representative: Tinel, Marie-Line

(57) **Abstract**

The invention relates to an ultra-low molecular weight heparin with an average molecular weight of 2000 to 3000 Daltons, an anti-FXa activity of about 160 U/mg and an anti-Flla activity of about 2 U/mg, in particular semuloparin, for use as a prophylactic antithrombotic agent in cancer patients receiving chemotherapy for locally advanced solid tumors, wherein said use is accompanied by an improval of the survival of patients with locally advanced cancer of the lungs or with a venous thromboembolism risk score equal to or greater than 3.

## Description

The invention relates to the use of semuloparin or a pharmaceutically acceptable salt thereof as a prophylactic antithrombotic agent in specific groups of patients receiving chemotherapy, wherein said use improves the survival of said patients.

Semuloparin, or AVE5026 (sanofi-aventis laboratory code), belongs to a new generation of hemisynthetic heparins. It is a new ultra-low molecular weight heparin (ULMWH), with an average molecular weight of 2000-3000 Daltons and a novel antithrombotic profile resulting from high anti-Factor Xa activity (-160 U/mg) and residual anti-Factor IIa activity (-2 U/mg). It is obtained by selective and controlled depolymerization of heparin by a phosphazene base, as described for example in Journal of Thrombosis and Haemostasis, 2009, vol. 7, 1143-1151.

Semuloparin, in the form of its sodium salt, is in clinical development for venous thromboembolism (VTE) prevention in cancer patients receiving chemotherapy (see for example G. Agnelli et al. in J. Clin. Oncol., 2011, vol. 29, suppl. abstr. LBA9014 and Hyer R. in Oncology Report, June 20, 2011).

The Applicant has shown, based on a large phase III trial, that semuloparin, when used for venous thromboembolism prophylaxis in cancer patients receiving chemotherapy, allows to improve the survival of some subgroups of patients, namely those having locally advanced cancer of the lungs and those having a VTE risk score equal to or greater than 3.

Therefore, the subject-matter of the invention is an ultra-low molecular weight heparin with an average molecular weight of 2000 to 3000 Daltons, an anti-FXa activity of about 160 U/mg and an anti-FIIa activity of about 2 U/mg, for use in improving the survival of patients with cancer receiving chemotherapy, said patients being selected in the group of patients having locally advanced cancer of the lungs and of patients having a VTE risk score equal to or greater than 3.

The anti-FXa and anti-FIIa activities described above are measured using amidolytic methods on a chromogenic substrate as adapted from the monograph on Low Molecular Weight Heparins (LMWHs) of the European Pharmacopeia in force, using as reconstitution buffer a tris-NaCl pH 7.4 buffer comprising PEG6000 (polyethylene glycol 6000) instead of albumin, and an ULMWH reference substance with an anti-FXa activity of 159 U/mg and an anti-FIIa activity of 2.9 U/mg. The potencies are expressed in units per mg due to the use of an internal ULMWH reference standard. Indeed, as a function of the concentration/dilution, lack of parallelism can be observed for anti-FIIa activities routine determination when the ULMWH is calibrated versus LMWH standard. The anti-FXa and anti-FIIa activities of the ULMWH reference substance described above have been determined relative to the international LMWH standard on a range of dilution where the parallelism was obtained. The results of the dosages are exploited according to §5.3 of the European Pharmacopeia in force ("Statistical analyses of dosages and biological assays results").

More particularly, the above ULMWH is semuloparin and the subject-matter of the invention is therefore semuloparin for use in improving the survival of patients with cancer receiving chemotherapy, said patients being selected in the group of patients having locally advanced cancer of the lungs and of patients having a VTE risk score equal to or greater than 3.

Said "VTE risk score" has been developed and validated for cancer patients receiving chemotherapy by Khorana A.A. et al. in Blood, 2008, vol. 111, 4902-7. According to such a risk score, the following patients are assigned a risk score of 3 or higher:
- patients with pancreatic or stomach cancer who have one or more of the risk factors below before initiating chemotherapy;
- patients with lung, bladder or ovarian cancer who have two or more of the risk factors below before initiating chemotherapy;
- patients with colon/rectum cancer who have three or more of the risk factors below before initiating chemotherapy.

Risk Factors:
- platelet count of 350 x 10⁹/L or more;
- hemoglobin less than 100 g/L or requiring red cell growth factors;
- leukocyte count more than 11 x 10⁹/L;
- BMI (body mass index) of 35 kg/m² or more.

In the groups of patients defined above, the ULMWH is administered as a prophylactic antithrombotic agent. The administration of semuloparin in such a prophylactic setting is accompanied, in the groups of cancer patients described above, by a beneficial effect on the survival of said patients.

In another embodiment, the subject-matter of the invention is therefore an ULMWH with an average molecular weight of 2000 to 3000 Daltons, an anti-FXa activity of about 160 U/mg and an anti-FIIa activity of about 2 U/mg, for use as an antithrombotic agent for the prophylaxis of venous thromboembolism in cancer patients receiving chemotherapy, wherein said use improves the survival of patients with locally advanced cancer of the lungs or of patients having a VTE risk score equal to or greater than 3.

The term "semuloparin", in the framework of the instant invention, encompasses any pharmaceutically acceptable salt thereof, in particular its sodium salt. The term "semuloparin" shall therefore be understood herein as "semuloparin or any pharmaceutically acceptable salt thereof".

In the framework of present invention, the terms below have the following meanings:
- "cancer patients" are defined as patients having malignant solid tumors and being under chemotherapy treatment. In the framework of the invention, said "cancer patients" may be ambulatory patients (outpatients) or hospitalized patients;
- "prophylaxis" refers to the administration of a therapy to an individual who is considered as being at risk for a thromboembolic pathology such as venous thromboembolism (including deep vein thrombosis, which may lead to pulmonary embolism), i.e. to an individual who does not already have symptoms of an established venous thromboembolic state;
- "venous thromboembolism" (VTE) designates the formation of a blood clot (named "thrombus") inside a blood vessel, obstructing the flow of blood through the circulatory system;
- "locally advanced" cancer or tumor: cancer/tumor which has spread from the site of the primary (original) tumor only to surrounding or nearby tissues or lymph nodes, but has not metastasized.

According to the instant invention, the improval of the survival status of the above-defined subgroups of cancer patients is demonstrated in a phase III clinical trial. A "phase III clinical trial" refers, in the framework of the instant invention, to a multinational, randomized, double-blinded study involving a large patients group (more than 3.000, as it will be described in details below), as defined by the health authorities and the regulatory laws and guidelines, aiming at being the definitive assessment of how effective and safe the drug is. According to the instant invention, and as it will be described in details below, said phase III clinical trial is performed in cancer patients, more specifically in cancer patients at high risk for VTE and who are undergoing chemotherapy for solid tumors.

In an embodiment of the invention, the use of the ULMWH with an average molecular weight of 2000 to 3000 Daltons, an anti-FXa activity of about 160 U/mg and an anti-FIIa activity of about 2 U/mg, as defined above, improves the survival rate of the patients.

In another embodiment of the invention, the use of the ULMWH with an average molecular weight of 2000 to 3000 Daltons, an anti-FXa activity of about 160 U/mg and an anti-FIIa activity of about 2 U/mg, as defined above, improves the survival duration of the patients, more particularly their median overall survival.

In an embodiment of the instant invention, the ULMWH is administered once daily.

Generally, administration of the ULMWH is started at the initiation of a course of chemotherapy and continued daily throughout any subsequent chemotherapy cycles for 3 months, or longer if the increased risk of VTE persists. In the phase III clinical trial of semuloparin in cancer patients described above, the median duration of treatment was 3.5 months.

In another embodiment of the invention, administration of the ULMWH is advantageously started at the initiation of a course of chemotherapy and continued once daily for 3 months. If chemotherapy is definitely discontinued, then treatment with the ULMWH should also be discontinued.

According to the instant invention, the patients to which the ULMWH is administered do not display severe renal impairment, which means that their estimated creatinine clearance (CLcr) value, calculated using the well-known Cockroft-Gault formula, shell not be less than 30 mL/min.

As used herein, the wording "an ULMWH ... for use as ..." shall be understood as being equivalent to the wording "use of an ULMWH for ..." or "use of an ULMWH for the preparation of a medicament for use in ...".

The invention therefore also relates to the use of an ULMWH as defined above for the manufacture of a medicament useful in improving the survival of patients with cancer receiving chemotherapy, said patients being selected in the group of patients having locally advanced cancer of the lungs and of patients having a VTE risk score equal to or greater than 3. All the embodiments and features described above also apply to said use.

The invention also relates to an article of manufacture comprising:
- a packaging material,
- a compound chosen from an ULMWH with an average molecular weight of 2000 to 3000 Daltons, an anti-FXa activity of about 160 U/mg and an anti-FIIa activity of about 2 U/mg, in particular semuloparin or a pharmaceutically acceptable salt thereof, and
- a label or package insert contained within said packaging material indicating that said compound improves the survival of patients with locally advanced cancer of the lungs and of patients having a VTE risk score equal to or greater than 3.

The invention also relates to a method for promoting the use of an ULMWH with an average molecular weight of 2000 to 3000 Daltons, an anti-FXa activity of about 160 U/mg and an anti-FIIa activity of about 2 U/mg, in particular semuloparin or a pharmaceutically acceptable salt thereof, the method comprising the step of conveying to a recipient at least one message chosen from:
- said ULMWH improves the survival of patients with locally advanced cancer of the lungs;
- said ULMWH improves the survival of patients having a VTE risk score equal to or greater than 3.

Having now described the present invention, the same will be more clearly understood by reference to the following examples of the invention, which are included herewith for purposes of illustration only and are not intended to be limiting of the invention.

The following abbreviations shall be used:
Cl: Confidence Interval
CIAC: Central Independent Adjudication Committee
CIFs: Cumulative Incidence Functions
CVC: central venous catheter
CT: computer tomography
DVT: deep vein thrombosis
IP: investigational product
PE: pulmonary embolism
UFH: unfractionated heparin
LMWH: low molecular weight heparin
OR: Odds Ratio
OS: Overall Survival
q.d.: *quaque die* (once daily)
(S)AEs: (serious) adverse events
s.c.: subcutaneously
vs.: *versus*
VTE: venous thromboembolism
95% (mid-p) Cl: 95% (mid-p) Confidence Interval

The SAVE-ONCO phase III study: A multinational, randomized, double blind, placebo-controlled study to evaluate the efficacy and safety of AVE5026 in the prevention of venous thromboembolism (VTE) in cancer patients at high risk for VTE and who are undergoing chemotherapy.

### 1) Study objectives

The primary objective of the study was to compare the efficacy of once daily (q.d.) subcutaneous (s.c.) injections of 20 mg AVE5026 with placebo in the prevention of VTE in cancer patients at high risk for VTE and who are undergoing chemotherapy.

The secondary objectives of the study comprised the evaluation of the safety of AVE5026 in cancer patients at high risk for VTE and who are undergoing chemotherapy, to document AVE5026 exposures and to assess the survival status in this population.

### 2) Study design

This was a multinational, multicenter, randomized, double-blind superiority study, with two parallel groups study.

The study was placebo-controlled, as a systematic venous thromboprophylaxis is neither recommended nor routinely used in patients undergoing chemotherapy and as there is no anticoagulant drug approved to date in this indication.

Cancer patients at high VTE risk, defined as patients with a metastatic or locally-advanced solid tumor of the lung, pancreas, stomach, colon/rectum, bladder or ovary and who are undergoing chemotherapy, were randomly assigned to receive once daily s.c. injection of either AVE5026 or placebo :
(i) until change in the initial chemotherapy regimen (i.e. addition or removal of at least one of the initial antineoplastic drugs) if this change occurred after the first 3 months of the study, or
(ii) at least 3 months and until the regimen ongoing at the 3-month time point is changed (change being defined as addition or removal of at least one of the antineoplastic drugs of the previous regimen) if the change in the initial chemotherapy regimen occurred within the first 3 months of the study and the patient continued on chemotherapy, or
(iii) until decision was made to stop definitely chemotherapy, if it occurred within the first three months of the study,
whichever came first.

Randomized treatment was allocated to eligible patients through a centralized randomization system using an Interactive Voice Response System (IVRS). In order to balance treatment groups with regard to prognostic factors and geographical region, a dynamic allocation was used taking into account three factors: the localization of the primary site of tumor (lung, pancreas, stomach, colon/rectum, bladder or ovary), the stage of the cancer (metastatic *versus* locally-advanced) and the geographical region (North America, South America, Western Europe, Eastern Europe, Asia and rest of the world). The randomization call occurred on the first day of the chemotherapy or the day after at the latest and as close as possible prior to the first IP injection.

During the study treatment period, visits were performed monthly ± 1 week (corresponding to scheduled chemotherapy visits). Signs and symptoms of VTE, bleeding, adverse events, specific concomitant medications (including chemotherapy) and compliance were assessed. Blood samples are drawn for laboratory tests and in all patients from selected centers for pharmacokinetic purposes.

At any time during the course of the study, if a patient experienced signs or symptoms evocative of VTE, unscheduled diagnostic test(s) were performed to confirm or rule out the presence of VTE. In addition, if a PE was discovered incidentally on a lung imaging test for tumor evaluation, a package was sent to the Blinded Adjudication Committee for review. All bleeding events and deaths reported up to the follow-up visit were adjudicated. In all these cases, the adjudication package consisted of relevant documentation such as all relevant films (venography or ultrasound for DVT, ventilation/perfusion lung scan, pulmonary angiogram or spiral CT lung scan for PE or any other imaging test leading to incidental VTE discovery) or autopsy report if available.

An end of treatment visit was done within 5 days after last study drug administration.

A follow-up visit was scheduled one month ± 1 week after the end of treatment visit. During this visit, information regarding adverse events (including bleedings and VTE) was collected.

In addition, survival status (alive, dead, or lost to follow up) was collected for all patients either one year after randomization or at the end of the study (i.e. 7 months following randomization of the last patient at the latest), whichever came first.

The duration of study participation per patient was variable and depended on the duration of chemotherapy. For a given patient, the duration of study period is the duration of study treatment followed by a one month follow-up period after the end of treatment visit. In addition, patients were screened within 3 weeks prior to the start of chemotherapy.

In any case, the study end date was at the latest seven months (6 months treatment period and one month follow-up) following the randomization of the last patient.

### 3) Selection of patients

### 3.1 : Inclusion criteria

Patients eligible for inclusion in the study were cancer patients :
- with metastatic or locally advanced solid tumor of the lung, pancreas, stomach, colon/rectum, bladder or ovary,
- planned to start a (new) course of chemotherapy with a minimum intent of 3 months therapy.

Inclusion in the study also required that the patients had signed informed consent.

Chemotherapy is herein defined as any conventional cytotoxic treatment. Biological agents used alone were not considered as chemotherapy, but could be associated with cytotoxic agents.

### 3.2 : Exclusion criteria

### 3.2.2 : Exclusion criteria related to study methodology

1. Legal lower age limitations (country specific).
2. Life expectancy less than 3 months.
3. ECOG (Eastern Cooperative Oncology Group) performance status of 3 or 4 (as published by Oken, M.M. et al. in Am. J. Clin. Oncol., 1982, vol. 5, pp. 649-655).
4. Calculated creatinine clearance < 30 mL/min according to Cockroft and Gault formula (Nephron, 1976, vol. 16, pp. 31-41), i.e. severe renal impairment.
5. Any major surgery (i.e. open surgery lasting more than 45 minutes from opening to closure) within the last 4 weeks or planned during the study treatment period.
6. Contra-indications to anticoagulation:
   - active or recent (<3 months) significant bleeding, including gastrointestinal bleeding or peptic ulcer;
   - history of bleeding disorder (congenital, acquired or unexplained repeated bleeding episodes);
   - uncontrolled arterial hypertension (systolic blood pressure > 180 mm Hg or diastolic blood pressure > 110 mm Hg);
   - hemorrhagic stroke or recent (in the last 3 months) brain, spinal or ophthalmic surgery;
   - known cerebral hemorrhagic lesion;
   - primary or metastatic tumor which is at high bleeding-risk according to investigator's judgment;
   - known structural damage or other pathologic process involving the central nervous system (brain metastases, vascular malformation ...);
   - thrombocytopenia (platelet count < 100 x 10⁹/l);
   - activated partial thromboplastin time (aPTT) > 1.5 ULN or International Normalized ratio (INR) > 1.5.
7. Any treatment with other anti-thrombotic agents within 2 weeks prior to randomization or planned during the study treatment period, such as:
   - parenteral anticoagulants (UFH, LMWH: enoxaparin, dalteparin, nadroparin..., or other agents such as fondaparinux, bivalirudin, hirudin);
   - oral anticoagulants (Vitamin K antagonists);
   - anti-GPIIb/IIIa (eptifibatide, tirofiban, abciximab);
   - thrombolytic agents.
   Notes:
   - Chronic treatment with anti-platelet agents such as low dose of aspirin (up to 325 mg/day) or clopidogrel or ticlopidine in patients with coronary artery disease is allowed.
   - To maintain patency of central venous catheter, saline flushing solutions are highly recommended. However, if it is local practice, flushing solutions with small amounts of heparin (max 500 units) are allowed.
   - Should an occlusion of the CVC occur, it is advised an attempt to restore the CVC patency by infusion of urokinase 5000-10000 units (could be repeated up to a dose of 50 000 units) or alteplase 2 mg.
8. Subject who requires a systematic venous thromboprophylaxis with anticoagulant or a curative anti-coagulant or thrombolytic treatment.
9. Pelvic venous obstruction or superior vena cava syndrome.
10. Subject unlikely to comply with protocol, e.g. uncooperative attitude, inability to return for follow-up visits, inability to receive daily injection (self-injection or by relative of patient or by health care professional) and unlikelihood of completing the study.
11. Treatment with any investigational product or investigational device in the last 30 days or 5 half lives (whichever is longer, if relevant) prior to randomization.
12. Any previous exposure to AVE5026 (e.g. participation in any previous AVE5026 clinical trial).

Note: A patient could not be randomized in the study more than once.

### 3.2.3 : Exclusion criteria related to AVE5026

13. History of heparin-induced thrombocytopenia.
14. Known hypersensitivity to UFH or LMWH.
15. Pregnant or nursing woman or women of childbearing potential not protected by highly effective contraceptive method of birth control as defined for contraception in the Informed Consent Form and/or in a local protocol addendum for the duration of the study and/or who are unwilling or unable to be tested for pregnancy.

### 4) Treatments

Patients were allocated to one of the two study treatments: AVE5026 or placebo of AVE5026.

The AVE5026 syringe contained 20 mg of AVE5026 in a 0.5 mL pre-filled syringe containing 0.4 mL of a sterile, isotonic solution with sodium chloride 0.9% and water for injection corresponding to a concentration of 50 mg/mL.

The matching placebo syringe was strictly identical in appearance, containing the same volume but without active component.

AVE5026 or its placebo was administered subcutaneously.

Study medication (AVE5026 or its placebo) started as close as possible after the randomization, which should take place as close as possible to the start of chemotherapy. The first injection was done at site under direct supervision. The investigator decided whether injections at home could be performed by the patient (self-injection) or by a relative, or whether it should be supported by a health care professional.

Study medication was administered once daily s.c. at approximately 24 hours apart. The time of the day was upon investigator's or patient's preference. However it was recommended to keep the same timing during the study.

### 5) Assessment of investigational product

### 5.1: Efficacy

The primary efficacy criterion was the time-to-first occurrence of any component of the composite endpoint of the following documented outcome results, confirmed by the CIAC (composed of thrombosis and bleeding experts, blinded to study medication assignment), from randomization up to 3 calendar days after last IP injection:
● Any symptomatic DVT of the lower limbs,
● Any symptomatic DVT of the upper limbs (including CVC-related thrombosis),
● Any non-fatal PE,
● VTE-related deaths (including fatal PE and unexplained deaths).

The VTE diagnosis needed to be confirmed or ruled out by objective investigations, described as follows.
● DVT of the lower limbs: the clinical diagnosis must be confirmed by compression ultrasound (CUS) or venography performed within 72 hours after the clinical suspicion. DVT was confirmed if the CUS was abnormal or if there was an intraluminal filling defect on the venography.
● DVT of the upper limbs: thrombosis of the central line was not considered a *priori* as a suspicion of DVT unless a patient presented symptoms such as arm swelling, erythema, pain, distal paresthesias, neck swelling, headache, and congestion of subcutaneous collateral veins. In any case, the clinical diagnosis must be confirmed by ultrasound (US) or venography performed within 72 hours after the clinical suspicion. DVT was confirmed if the US is abnormal or if there was an intraluminal filling defect on the venography.
● Pulmonary embolism: the clinical diagnosis must be confirmed by ventilation /perfusion lung scan, pulmonary angiogram or spiral Computer Tomography (CT) lung scan within 72 hours after the clinical suspicion. PE was confirmed in case of:
   - intraluminal filling defect in (sub)segmental or more proximal branches on a spiral CT scan, or
   - intraluminal filling defect on the pulmonary angiogram, or
   - a perfusion defect of at least 75% of a segment with a local normal ventilation result (high-probability) on ventilation/perfusion lung scan, or
   - an inconclusive spiral CT, pulmonary angiography or lung scintigraphy with demonstration of DVT in the lower extremities by compression ultrasound or venography, or
   - a fatal PE based on autopsy.

The patient population used in the analysis of the primary efficacy endpoint (i.e. primary efficacy population) was the Intent-To-Treat (ITT) population, which included all randomized patients. Patients were analyzed in the treatment group to which they were allocated by the IVRS (i.e. "as randomized" regardless of treatment actually received).

### 5.2: Safety

The safety analysis period was defined as the period from the first IP injection up to the last IP injection plus 3 calendar days (called "on-treatment period"). The safety population was defined as all randomized patients exposed to the study medication, regardless of the amount of treatment administered.

Clinical safety was assessed by bleedings (major bleeding and clinically relevant non-major bleeding), vital signs, transfusions requirement, hemoglobin, platelet count, liver and renal laboratory data, (S)AEs and deaths (classified as VTE-related death, fatal bleeding or other by a blinded Adjudication Committee) up to 3 calendar days after last IP injection and up to the follow-up visit.

The definition of major bleeding was in agreement with the International Society on Thrombosis and Haemostasis (ISTH) recommendation for clinical investigations of antihemostatic products in non-surgical patients (J. Thromb. Haemost., 2005, vol. 3, pp. 692-4), namely:
● Fatal bleeding, and/or
● Symptomatic bleeding in a critical area or organ, such as intracranial, intraspinal, intraocular, retroperitoneal, intraarticular or pericardial, or intramuscular with compartment syndrome, and/or
● Bleeding causing a fall in hemoglobin level of 2 g/dL (1.24 mmol/L) or more, or leading to transfusion of two or more units of whole blood or red cells

Overt bleedings requiring a medical intervention and not meeting any of the criteria for major bleeding were classified as "clinically relevant non-major". Bleedings events others than major or clinically relevant non-major bleedings were classified as non-clinically relevant bleedings.

### 6) Results concerning study patients and treatment

A total of 3212 patients were randomized in the study, 1608 in the semuloparin group and 1604 in the placebo group. Amongst the randomized patients, 68.2% had a metastatic cancer and 31.8% had a locally advanced cancer.

The median duration of study treatment was around 3.5 months in the two groups, with most of patients receiving 3 to 6 months of study treatment: 48.8% in the semuloparin group and 48.3% in the placebo group.

### 7) Efficacy results

The primary analysis of the primary endpoint consisted of the comparison of the two treatment groups (AVE5026 and placebo) using the two-sample test of Gray for comparing Cumulative Incidence Functions (CIFs), at a significant level of 0.05 (2-sided). An estimation of the treatment effect (hazard ratio and 95% Cls) was provided using Fine and Gray regression model for CIFs.

CIFs were estimated separately for the two treatment groups with Prentice non-parametric estimator using a model of cause-specific hazards; corresponding 95% 2-sided Cls were computed by Keiding and Andersen formula with variance computed using the delta method.

The event rates of the primary efficacy endpoint (any VTE) and its components occurring during the efficacy analysis period were summarized by treatment group.

Table 1 describes the incidences of the primary efficacy endpoint of SAVE-ONCO study by treatment groups. Table 2 describes the results for each component of the primary efficacy endpoint and table 3 describes the key subgroups analyses by cancer stage and location of primary tumor.

**Table 1: Any VTE or VTE-related death during the efficacy analysis period (time to first VTE event) - Primary efficacy analysis - Intent-To-Treat population**

| | AVE5026 | Placebo |
|---|---|---|
| | (N = 1608) | (N = 1604) |
| Any VTE or VTE-related death [n (%)] Comparison *versus* placebo: | 20 (1.2%) | 55 (3.4%) |
| Hazard ratio (95% Cl) | | 0.36 (0.21 to 0.60) |
| Gray test p-value | | < 0.0001 |
| Number of competing events * [n (%)] | 78 (4.9%) | 74 (4.6%) |

| | | |
|---|---|---|
| N: number of patients in the primary efficacy population n: number of patients showing a given event * : Competing events considered deaths from cause other than VTE occurring during the efficacy analysis period. | | |

**Table 2: Components of the primary efficacy endpoint**

| | AVE5026 | Placebo | HR |
|---|---|---|---|
| | n/N (%) | n/N (%) | (95% mid-p Cl) |
| Any VTE or VTE-related death | | | |
| n/N (%) | 20/1608 (1.2%) | 55/1604 (3.4%) | 0.36 |
| 95% mid-p Cl | (0.8 to 1.9) | (2.6 to 4.4) | (0.21 to 0.60) |
| Symptomatic DVT | | | |
| n/N (%) | 11/1608 (0.7%) | 34/1604 (2.1%) | 0.32 |
| 95% mid-p Cl | (0.4 to 1.2) | (1.5 to 2.9) | (0.15 to 0.62) |
| Symptomatic DVT of the upper limbs | | | |
| n/N (%) | 3/1608 (0.2%) | 9/1604 (0.6%) | 0.33 |
| 95% mid-p Cl | (0.0 to 0.5) | (0.3 to 1.0) | (0.07 to 1.18) |
| Symptomatic DVT of the lower limbs | | | |
| n/N (%) | 8/1608 (0.5%) | 25/1604 (1.6%) | 0.32 |
| 95% mid-p Cl | (0.2 to 0.9) | (1.0 to 2.3) | (0.13 to 0.69) |
| Proximal DVT of the lower limbs | | | |
| n/N (%) | 4/1608 (0.2%) | 19/1604 (1.2%) | 0.21 |
| 95% mid-p Cl | (0.1 to 0.6) | (0.7 to 1.8) | (0.06 to 0.58) |
| Distal DVT of the lower limbs | | | |
| n/N (%) | 4/1608 (0.2%) | 12/1604 (0.7%) | 0.33 |
| 95% mid-p Cl | (0.1 to 0.6) | (0.4 to 1.3) | (0.09 to 0.99) |
| PE | | | |
| n/N (%) | 10/1608 (0.6%) | 24/1604 (1.5%) | 0.41 |
| 95% mid-p Cl | (0.3 to 1.1) | (1.0 to 2.2) | (0.20 to 0.86) |
| Non-fatal PE | | | |
| n/N (%) | 3/1608 (0.2%) | 15/1604 (0.9%) | 0.20 |
| 95% mid-p Cl | (0.0 to 0.5) | (0.5 to 1.5) | (0.05 to 0.63) |
| Any VTE-related death | | | |
| n/N (%) | 7/1608 (0.4%) | 9/1604 (0.6%) | 0.77 |
| 95% mid-p Cl | (0.2 to 0.9) | (0.3 to 1.0) | (0.27 to 2.13) |

**Table 3: Any VTE or VTE-related death during the efficacy analysis period (time to first VTE event) - Key subgroups analyses - Intent-To-Treat population**

| | AVE5026 | Placebo | Hazard ratio |
|---|---|---|---|
| | n/N (%) | n/N (%) | (95% Cl) |
| Stage of cancer: | | | |
| metastatic | 16/1097 (1.5%) | 38/1095 (3.5%) | 0.42 (0.23 to 0.75) |
| locally advanced | 4/511 (0.8%) | 17/509 (3.3%) | 0.23 (0.08 to 0.68) |
| Location site of primary tumor: | | | |
| Lung | 9/951 (1.5%) | 25/589 (4.2%) | 0.36 (0.17 to 0.77) |
| Pancreas | 3/126 (2.4%) | 14/128 (10.9%) | 0.22 (0.06 to 0.76) |
| Stomach | 1/204 (0.5%) | 4/207 (1.9%) | 0.25 (0.03 to 2.20) |
| Colon/rectum | 5/464 (1.1 %) | 9/461 (2.0%) | 0.54 (0.18 to 1.60) |
| Bladder | 1/32 (3.1%) | 3/31 (9.7%) | 0.30 (0.03 to 2.95) |
| Ovary | 1/191 (0.5%) | 0/188 | NA |

| | | | |
|---|---|---|---|
| NA: not applicable | | | |

These tables demonstrate a significant superiority of semuloparin versus placebo, with a consistent effect over the individual components of the primary efficacy endpoint, DVT and PE.

As apparent from tables 1 and 2, risk reduction compared to placebo was 64% for any VTE or VTE-related death (primary endpoint), 68% for DVT (67% for DVT of the upper limbs and 68% for DVT of the lower limbs) and 59% for PE.

The additional analyses amongst subgroups of the Intent-To-Treat population (table 3) show that no heterogeneity of effect was detected across stage of cancer (metastatic or locally advanced) (interaction p-value = 0.3236) and across location site of primary tumor (lung, pancreas, stomach, colon/rectum, bladder or ovary) (interaction p-value = 0.7994).

### 8) Safety results

CIFs were estimated separately for the two treatment groups together with 95% 2-sided Cls. An estimation of the treatment effect (hazard ratio and 95% Cls) was given using Fine and Gray regression model for CIFs.

Table 4 describes the incidence of any treatment emergent clinically relevant bleeding, major bleeding and clinically relevant non-major bleeding in the safety population of the SAVE-ONCO study.

**Table 4: Number of patients with treatment emergent clinically relevant bleedings, major bleedings and non-major bleedings - Safety population**

| | AVE5026 | Placebo | OR |
|---|---|---|---|
| | (N=1589) | (N=1583) | (95% mid-p Cl) |
| Any clinically relevant bleeding: | | | |
| n (%) | 45 (2.8%) | 32 (2.0%) | 1.41 |
| 95% mid-p Cl | (2.1 to 3.7) | (1.4 to 2.8) | (0.89 to 2.25) |
| Any major bleeding: | | | |
| n (%) | 19 (1.2%) | 18 (1.1%) | 1.05 |
| 95% mid-p Cl | (0.7 to 1.8) | (0.7 to 1.8) | (0.55 to 2.04) |
| Any clinically relevant non-major bleeding: | | | |
| n (%) | 26 (1.6%) | 14 (0.9%) | 1.86 |
| 95% mid-p Cl | (1.1 to 2.4) | (0.5 to 1.4) | (0.98 to 3.68) |

| | | | |
|---|---|---|---|
| N: number of patients in the safety population n: number of patients with bleedings | | | |

This table shows that the incidence of clinically relevant bleedings, including clinically relevant non-major bleedings, is slightly higher in the semuloparin group vs. placebo, and is consistent with what is expected for an antithrombotic product. However, the incidence of major bleedings is similar between the two studied groups (OR = 1.05, 95% mid-p Cl [0.55 to 2.04]).

### 9) Survival results

Tables 5 and 6 describe the subgroup analyses on survival (survival rate on table 5, median overall survival on table 6), based on stage of cancer. These tables show a favourable trend for semuloparin on the survival rate of patients with locally advanced cancer.

**Table 5: Number of deaths - Subgroup analysis - Stratification factors - Intent-To-Treat population**

| | Number of deaths n/N (%) | | Hazard ratio (95% Cl) |
|---|---|---|---|
| | AVE5026 | Placebo | |
| Stage of cancer: | | | |
| - metastatic | 517/1097 (47.1 %) | 514/1095 (46.9%) | 1.00 (0.89 to 1.13) |
| - locally advanced | 181/511 (35.4%) | 200/509 (39.3%) | 0.86 (0.70 to 1.05) |
| p-value of interaction | | | 0.2042 |

| | | | |
|---|---|---|---|
| N: number of patients in the Intent-To-Treat population n: number of deaths | | | |

**Table 6: Median overall survival (months) - Subgroup analysis - Stratification factors - Intent-To-Treat population**

| | Median OS | |
|---|---|---|
| | Months (95% Cl) | |
| | AVE5026 | Placebo |
| Stage of cancer: | | |
| - metastatic | 12.1 (11.83 to 12.68) | 12.1 (11.43 to 12.68) |
| - locally advanced | 13.9 (12.78 to 20.24) | 14.2 (12.58 to 16.13) |

Analysis of the overall survival in the subgroup of patients with locally advanced cancer was carried out according to the location of the primary tumor (tables 7 and 8). In the subgroup with adequate presentation, namely the lung cancer subgroup (491 patients), a benefit of semuloparin on survival is observed: the death rate decreases from 49.8% (124/249) on placebo to 41.7% (101/242) on semuloparin, and the median overall survival increases from 11.2 months on placebo to 12.7 months on semuloparin.

**Table 7: Subgroups analysis - Number of deaths - Kaplan Meier survival estimates by location site of primary tumor - Intent-To-Treat population - Patients with locally advanced cancer**

| | Number of deaths n/N (%) | | Hazard ratio (95% Cl) |
|---|---|---|---|
| | AVE5026 | Placebo | |
| Locally advanced cancer of: | | | |
| - lung | 101/242 (41.7%) | 124/249 (49.8%) | 0.76 (0.58 to 0.99) |
| - pancreas | 29/48 (60.4%) | 20/43 (46.5%) | 1.31 (0.73 to 2.32) |
| - stomach | 17/48 (35.4%) | 22/48 (45.8%) | 0.54 (0.28 to 1.06) |
| - colon/rectum | 19/83 (22.9%) | 18/84 (21.4%) | 1.14 (0.60 to 2.18) |
| - bladder | 1/9 (11.1%) | 1/7 (14.3%) | 0.72 (0.04 to 11.54) |
| - ovary | 14/81 (17.3%) | 15/78 (19.2%) | 0.98 (0.47 to 2.04) |

| | | | |
|---|---|---|---|
| N: number of patients in the Intent-To-Treat population n: number of deaths | | | |

**Table 8: Subgroups analysis - Overall survival (months) - Kaplan Meier survival estimates by location site of primary tumor - Intent-To-Treat population - Patients with locally advanced cancer**

| | Median OS | |
|---|---|---|
| | Months (95% Cl) | |
| | AVE5026 | Placebo |
| Locally advanced lung cancer | 12.7 (11.47 to 13.93) | 11.2 (10.05 to 12.58) |

Analysis of the numbers of deaths and of the overall survival according to the VTE risk score of the patients is detailed table 9. The results in table 9 show the benefit of the treatment with semuloparin on survival, both in terms of number of deaths and of median overall survival, in the subgroup of cancer patients with a VTE risk score ≥ 3.

**Table 9: Subgroups analysis - Number of deaths and overall survival (months) according to VTE risk score - Intent-To-Treat population**

| | VTE risk score | | |
|---|---|---|---|
| | 0 | 1-2 | ≥ 3 |
| AVE5026: | | | |
| - Number of deaths [n/N (%)] | 92/313 (29.4%) | 442/995 (44.4%) | 153/271 (56.5%) |
| - Median OS [months (95% Cl)] | 15.0 (13.31 to NC) | 12.6 (11.99 to 12.91) | 9.2 (7.98 to 11.76) |
| Placebo: | | | |
| - Number of deaths [n/N (%)] | 83/301 (27.6%) | 443/1003 (44.2%) | 181/279 (64.9%) |
| - Median OS [months (95% Cl)] | 19.9 (14.16 to NC) | 12.6 (11.99 to 13.73) | 6.9 (5.65 to 8.51) |
| HR (95% Cl) | 1.13 (0.84 to 1.53) | 1.00 (0.88 to 1.14) | 0.75 (0.61 to 0.93) |
| p-value of interaction | 0.0322 | | |

| | | | |
|---|---|---|---|
| NC: non communicated | | | |

## Claims

1. An ultra-low molecular weight heparin with an average molecular weight of 2000 to 3000 Daltons, an anti-FXa activity of about 160 U/mg and an anti-FIIa activity of about 2 U/mg, for use in improving the survival of patients with cancer receiving chemotherapy, said patients being selected in the group of patients having locally advanced cancer of the lungs and of patients having a venous thromboembolism risk score equal to or greater than 3.

2. The ultra-low molecular weight heparin according to claim 1, wherein said ultra-low molecular weight heparin is semuloparin or any pharmaceutically acceptable salt thereof.

3. The ultra-low molecular weight heparin according to claim 1 or 2, for use in improving the survival rate of said patients.

4. The ultra-low molecular weight heparin according to any of claims 1 to 3, for use in improving the survival duration of said patients.

5. The ultra-low molecular weight heparin according to any of claims 1 to 4, for use in improving the median overall survival of said patients.
